# EUROPEAN PATENT SPECIFICATION

(11) **EP 1 315 958 B1**
(45) Date of publication and mention of the grant of the patent: **14.03.2007**
(21) Application number: 99915053.5
(22) Date of filing: 26.03.1999
(51) Int. Cl.: C12N 1/19, C12N 15/64, C12N 15/81, G01N 33/50

(54) **Method for detecting protein-protein interaction**
Untersuchungsverfahren für Protein-Protein-Interaktion
Procédé pour la détection de l'interaction protéine-protéine

(30) Priority: 26.03.1998 US 79480 P; 27.03.1998 US 49325
(43) Date of publication of application: 04.06.2003
(73) Proprietor: GLAXO GROUP LIMITED, Greenford, Middlesex UB6 ONN (GB)
(72) Inventor: WEINER, Michael Phillip, Research Triangle Park, NC 27709 (US); BUCKHOLZ, Richard Gordon, Research Triangle Park, NC 27709-3398 (US)
(74) Representative: Learoyd, Stephanie Anne
(86) International application number: PCT/US1999/006671
(87) International publication number: WO 1999/049294

(56) References cited:
- FINLEY R L ET AL: "TWO-HYBRID ANALYSIS OF GENETIC REGULATORY NETWORKS. THE YEAST TWO-HYBRID SYSTEM" YEAST TWO-HYBRID SYSTEM ( ADVANCES IN MOLECULAR BIOLOGY ), XX, XX, 1997, pages 197-214, XP002922642
- FINLEY R L ET AL: "INTERACTION MATING REVEALS BINARY AND TERNARY CONNECTIONS BETWEEN DROSOPHILA CELL CYCLE REGULATORS" PROCEEDINGS OF THE NATIONAL ACADEMY OF SCIENCES OF USA, NATIONAL ACADEMY OF SCIENCE. WASHINGTON, US, vol. 91, 1 December 1994 (1994-12-01), pages 12980-12984, XP002047989 ISSN: 0027-8424
- BUCKHOLZ RICHARD G ET AL: "Automation of yeast two-hybrid screening" JOURNAL OF MOLECULAR MICROBIOLOGY AND BIOTECHNOLOGY, vol. 1, no. 1, August 1999 (1999-08), pages 135-140, XP008038784 ISSN: 1464-1801
- P. L. BARTEL, ET AL.: 'The Yeast Two-Hybri System', 1997, OXFORD UNIVERSITY PRESS, NEW YORK, USA, ISBN 0-19-510938-4 Artikel R. L. FINLEY, ET AL.: 'Two-Hybrid Analysis of Genetic Regulatory Networks', XP002922642.

## Description

### FIELD OF THE INVENTION

The present invention relates to methods useful for detecting protein-protein interactions. Protein-protein interactions enable the association of two or more proteins through the formation of non-covalent bonds when two protein surfaces are precisely matched. These bonds account for the specificity of recognition. Protein-protein interactions are involved, for example, in the assembly of enzyme subunits; in antigen-antibody reactions; in forming the supramolecular structures of ribosomes, filaments, and viruses; in transport; and in the interaction of receptors on a cell with growth factors and hormones. Products of oncogenes can give rise to neoplastic transformation through protein-protein interactions.

### BACKGROUND OF THE INVENTION

The yeast two-hybrid (Y2H) assay is a method for detecting protein-protein interactions using a genetic system. The technique may be used for charting protein interactions, and hence, identifying potential partners in genetic pathways. The assay is sensitive and yields the DNA sequences encoding proteins that interact. In a typical two-hybrid assay, a known protein that forms part of a DNA-binding domain hybrid is assayed against a library of all possible proteins present as transcriptional activation domain hybrids. Some two hybrid approaches rely on interaction mating. In this method, the protein fused to the DNA-binding domain and the protein fused to the activation domain are expressed in two different haploid yeast strains of opposite mating type and the strains are mated to determine if the two proteins interact. When haploid yeast strains of opposite mating type come into contact, mating occurs and results in fusion of the two haploids to form a diploid yeast strain. An interaction can thus be determined by measuring activation of a two-hybrid reporter gene in the dipoid strain.

Finley R . L. et al. (1997) describe methods for performing interaction mating assays on small or large sets of proteins using the interaction trap in which a plate or bait strains and a plate of prey strains are each pressed to the same replica velvet and the impression is lifted with a plate containing YPD medium ("Two-Hybrid Analysis of Genetic Regulatory Networks" Advances in Molecular Biology (1997) pages 197-214). Finley R. L. et al. (1994) describe interactions between Drosophila melanogaster cell cycle regulatory proteins by a yeast interaction-mating technique that extends the interaction trap (a yeast two-hybrid method) and involves displaying the pattern of interactions in interaction matrices, two-dimensional arrays of diploid strains on appropriate indicator plates (Proceedings of the National Academy of Sciences of USA National Academy of Science, Washington, 91:12980-12984)."

WO 94/10300 and U.S. Patent No. 5,283,173 describe methods for detecting the interaction between proteins using reconstitution of the activity of a transcriptional activator. This reconstitution makes use of chimeric genes which express hybrid proteins. The first hybrid contains the DNA-binding domain of a transcriptional activator fused to a known protein (the "bait"), with the DNA binding domain DNA binding element placed upstream of a reporter gene. "Prey" proteins are cloned as either random sequences or cDNAs and are fused to the amino- or carboxy-terminus of a transcription activation domain. If the bait and prey proteins are able to interact, they bring into close proximity the two domains of the transcriptional activator. This proximity is sufficient to cause transcription, which can be detected by the activity of a reporter gene that contains a binding site for the DNA-binding domain.

The disadvantages of these techniques is that irrelevant interactions with yeast proteins are generated. These include false-positive interactions that are unlikely to be found in living cells, and false-negative interactions, that is, those interactions that would otherwise be detected but are not. The techniques as disclosed in WO 94/10300 and U.S. Patent No. 5,283,173 require the use of mating in solid medium which is cumbersome, labor-intensive, and does not preserve diploid cells for further analysis.

We have developed the mating strategy of the yeast two-hybrid assay into an automated format which allows many bait proteins to be processed. The format uses an arraying means, for example, microtiter plates and liquid mass-mating of a subset of a large, complex library. By tracking positive interactions in the library, we have also developed a method to create a functionally-subtracted library, that is, one that can be made devoid of a scorable phenotype. For example, our method allows for the determination of detection of hybrids that react promiscuously with many targets, such as heat shock proteins, and their elimination from any future considerations.

### SUMMARY OF THE INVENTION

In accordance with the present invention there is provided a method for detecting protein-protein interactions comprising liquid mass-mating of subsets of a large, complex library. The method provides a means for subtracting irrelevant protein-protein interactions to yield a "functionally-subtracted" assay.

### DETAILED DESCRIPTION OF THE INVENTION

According to an aspect of the present invention, there is provided a method for detecting an interaction between a first test protein and a second test protein, comprising:
(a) providing a host cell containing a reporter gene wherein the reporter gene expresses a detectable protein when the reporter gene is activated by an amino acid sequence including a transcriptional activation domain when the transcriptional activation domain is in sufficient proximity to the reporter gene;
(b) providing a first chimeric gene that is capable of being expressed in the host cell, the first chimeric gene comprising a DNA sequence that encodes a first hybrid protein, the first hybrid protein comprising:
   (i) a DNA-binding domain that recognizes a binding site on the reporter gene in the host cell; and
   (ii) a first test protein or fragment thereof that is to be tested for interaction with at least one second test protein or fragment thereof;
(c) providing a second chimeric gene that is capable of being expressed in the host cell, the second chimeric gene comprising a DNA sequence that encodes a second hybrid protein, the second hybrid protein comprising:
   (i) the transcriptional activation domain; and
   (ii) a second test protein or fragment thereof that is to be tested for interaction between the first test protein or fragment thereof; wherein interaction between the first test protein and the second test protein in the host cell causes the transcriptional activation domain to activate transcription of the reporter gene;
(d) introducing the second chimeric gene into the host cell and subsequently introducing said cells into an arraying means thereby creating a master library plate;
(e) introducing cells from the master library plate into a second arraying means which is an arraying means for liquid media thereby creating a mating set;
(f) introducing the first chimeric gene into the host cell and subsequently introducing said cell into the mating set thereby allowing mating to occur in liquid medium;
(g) selecting for outgrowth of the interaction of the first and second genes;
(h) removing a portion of the mating set to a third arraying means thereby creating a rescue set;
(i) determining whether the reporter gene has been expressed in the mating set; and
(j) analyzing the cells from the rescue plate.

The term "reporter gene" or "marker gene" as used herein means any gene whose expression may be assayed. More than one reporter gene may be encoded by the host cell in step (a) above.

The term "arraying means" as used herein means any method for holding clones in liquid media, suspension, or solid media, for example, microtiter plates or test tubes.

The term "selecting for outgrowth" as used herein means any method using a selectable means to either amplify or isolate a set of interacting proteins. This selectable means may include outgrowth in a nutritionally-deficient growth medium wherein the interacting proteins cause transcription of a biosynthetic gene or pathway. Examples of other useful selectable means include amino acid, metabolic, catabolic and nucleic acid biosynthetic genes, such as yeast HIS3, URA3, and LYS2, GAL1, *E. coli* galK, and CAT, GUS, antibiotic resistance, and any gene encoding a cell surface antigen for which antibodies are available. Outgrowth may be allowed to proceed for 5 - 10 days prior to selecting for outgrowth.

The term "analyzing" as used herein means any method for obtaining information regarding protein-protein interactions, for example, selecting positive clones, performing PCR, DNA sequence analysis, and comparison with databases such as LifeSeq® (Incyte Pharmaceuticals) or Genbank.

The term "functionally substracted" means devoid of a detectable phenotype that represents an irrelevant protein-protein interaction.

In a further aspect of the invention, determination of reporter gene expression and analysis of cells may be accomplished in one step, that is steps (i) and (j) above may be combined. Alternatively, steps (h), (i), and (j) may be eliminated.

A yeast host strain may be engineered to express the protein (the "bait") of therapeutic or diagnostic interest as a fusion protein covalently bonded to a known DNA binding domain of a transcriptional activator.
The yeast host strain also contains one or more "reporter genes", that is genes whose transcription is detected in response to a bait-prey interaction. Bait proteins, via their DNA binding domain, bind to their specific DNA site upstream of a reporter gene; reporter gene transcription is not stimulated, however, because the bait protein lacks its own activation domain.

To isolate genes encoding novel interacting proteins, cells of this strain containing a reporter gene and expressing a bait protein are transformed with individual members of a DNA (for example, a cDNA) expression library. Each member of the library directs the synthesis of a candidate interacting protein fused to a weak and invariant gene activation domain tag. The library-encoded proteins ("prey" proteins) that physically interact with the promoter-bound bait protein detectably activate the transcription of the downstream reporter gene and provide a ready assay for identifying particular cells which harbor a DNA clone encoding an interacting protein of interest.

In one embodiment, a cDNA library, created in *E. coli,* and comprising cDNA fused to the DNA sequence encoding the activation domain of the transcriptional activator, GAL4 protein, is plated onto 960 LB agar plates at a density of 1000 clones per plate. The *E. coli* colonies on each plate are pooled, plasmid DNAs are isolated, and the DNAs are used to transform yeast. The transformed yeast are plated onto solid medium and the colonies on each plate are pooled and aliquoted to separate wells of a 96-well microtiter plate to create an arrayed set of 10 "master library" plates. Five microliters from each well of the master library set is re-aliquoted to create a "mating set" and 5 µl of bait-containing yeast is then added separately to each well. The "bait" comprises a chimeric gene that expresses a hybrid protein containing the DNA-binding domain of GAL4 fused to a known protein. The host yeast strain contains the GAL1-lac-Z gene, which is able to bind the GAL4 DNA-binding domain. The GAL1-lacZ gene contains the *E. coli* lacZ gene encoding β-galactosidase. The activity of β-galactosidase is a measure of GAL4 function. Growth of yeast on galactose requires the transcription of genes regulated by GAL4 and is also a measure of GAL4 function. The liquid mass-mating is allowed to proceed for a period of time and the mating mixture is diluted 100 fold with leucine drop-out medium. After outgrowth of positively-interacting mated yeast diploids in the drop-out medium, a portion is removed to a separate set of "rescue" plates and a βGal analysis is performed on the mating set. Transcriptional activation can be determined by measuring β-galactosidase activity on galactose containing media. Wells containing any βGal activity are identified and clones from the corresponding set of wells from the rescue plates are analyzed by PCR sequencing.

Irrelevant protein-protein interactions may be eliminated by recombining only productive clones to the master library, thereby eliminating clones that produce proteins that are known to interact with many other proteins, for example heat shock-proteins.

There is also provided a method for an open reading frame cloning strategy that involves the dynamic recoding of the ends of DNA molecules. This cloning strategy increases the efficiency of the assay by eliminating from analysis all clones that encode proteins that are out of frame with respect to the activation domain.

In dynamic recoding of an activation domain, the 3' end of the activation domain gene may be recoded to incorporate an amino acid hybrid peptide that also encodes the DNA controlling elements necessary for *E.coli* gene expression. In one aspect, these controlling elements comprise, in series; i) a sequence, for example, -35 and a -10 sequence, which acts as an *E.coli* promoter to initiate mRNA transcription, ii) a ribosome binding site and an ATG fMet codon necessary to initiate protein translation, iii) a multiple cloning site composed of one or more restriction sites which are preferably unique to the cloning vector, into which to clone stuffer fragments of DNA that can encode protein fusions to the activation domain, and iv) a reporter gene, for example, the lacZ gene, cloned out-of frame with respect to the ATG codon. In the open reading frame cloning system, the ATG may be in-frame with respect to the activation domain, the ATG may be out-of-frame with respect to the lacZ gene, there is a neglegible amount of βGal protein produced by the host cell in the absence of a stuffer fragment that restores the lacZ gene reading frame, and there is an absence of termination codons the end of the activation domain gene and the ATG codon.

The term "stuffer fragments" means any fragment of DNA generated synthetically, or through use of a method generally used to generate random or 3'-end primed cDNA molecules that can be cloned into the multiple cloning site of the above open reading frame cloning system.

In one aspect, random primed cDNA used as stuffer fragments may be sized-selected by agarose or polyacrylamide gel electrophoresis. Individual cDNA sized-selected by gel electrophoresis or other means may contain fragments which when cloned into the vector system described may be in one of six reading frames (3 reading frames in both forward and reverse orientation).

The recoded activation domain may be used in conjunction with the out-of-frame reporter gene to select for clones that restore the reading frame of the reporter gene. For example, if the lacZ gene is initially out-of-frame with respect to the ATG start of the recoded portion of the activation domain, then clones that restore the reading frame between that ATG and the lacZ gene will create protein fusions of that clone to the lacZ gene product. Fusions that restore βgal activity may be selected for chromogenically by using well-known dyes (e.g., Xgal) or on selective growth media containing lactose as the sole carbon source.

In the open reading frame cloning system, an *E.coli* suppressible termination codon (for example a TAG amber termination codon) may be encoded between the stuffer fragment and the reporter gene such that in phenotypically suppressing *E.coli* host strains the stop codon is suppressed by a suppressing tRNA molecule that inserts a specific amino acid. In non-suppressing host cells in which the interaction assay is performed the termination of protein translation would occur at the termination codon. The advantage to having this suppressible system is that the open reading frame reporter protein will not be fused to the carboxyl terminus of the encoded stuffer fragment-activation domain hybrid protein.

The host cell is a yeast cell, advantageously *Saccharomyces cerevisiae.*

The bait protein may be derived from a bacterial protein, a viral protein, an oncogene-encoded protein, a growth factor or an enzyme. Bait proteins may be chosen from any protein of known or suspected diagnostic or therapeutic importance. Preferred bait proteins include oncoproteins (such as myc, ras, src, fos) or any other proteins involved in cell cycle regulation (such as kinases, phosphatases).

Prey proteins may be encoded on a library of plasmids containing DNA inserts that are derived from genomic DNA, cDNA, or synthetically generated DNA sequences fused to the DNA sequence encoding the second amino acid domain. cDNAs may be constructed from any mRNA population and inserted into an equivalent expression vector. Such a library of choice may be constructed *de novo* using commercially available kits (for example, from Stratagene, La Jolla, CA) or using well established preparative procedures (for example, Current Protocols in Molecular Biology, New York, John Wiley & Sons, 1987). Alternatively, a commercially available cDNA library may be used. A prey protein may be encoded by a synthetic sequence or may be the product of a randomly generated open reading frame or a portion thereof.

Any suitable reporter gene may be used, for example, LEU2 gene or the lacZ gene. Examples of other useful genes whose transcription can be detected include amino acid and nucleic acid biosynthetic genes, such as yeast HIS3, URA3, and LYS2, GAL1, *E. coli* galK, GFP, CUP1, and CAT, GUS, antibiotic resistance, and any gene encoding a cell surface antigen for which antibodies are available.

Those skilled in the art will also recognize that the reporter gene, DNA binding domain, and gene activation domain components may be derived from any appropriate eukaryotic or prokaryotic cell genomes or cDNAs as well as artificial sequences.

Plasmid constructs, transformation, transfection, cell culture and detection of transcription may be performed by any method known in the art, for example, U.S. Patent No. 5,283,173 and WO 94/10300.

Any means for introducing genes into host cells may be used, for example, electroporation, transfection, transformation, or mating.

Advantages of the described invention include increased efficiency through elimination from futher analysis of promiscuous proteins in arrayed libraries, creation of a means to functionally subtract classes of proteins from libraries, elimination from further analysis of clones not in a sepcified reading frame, reduced labor over current methods, re-use of primary libraries from arrayed master library sets, and accumulated knowledge over time of the make-up of the arrayed clones.

The invention may be illustrated by the following non-limiting examples.

### EXAMPLE 1

### Liquid mass-mating, functionally-subtracted yeast two-hybrid assay

Restriction and DNA modification enzymes were purchased from various manufacturer's and used according to their recommendations.

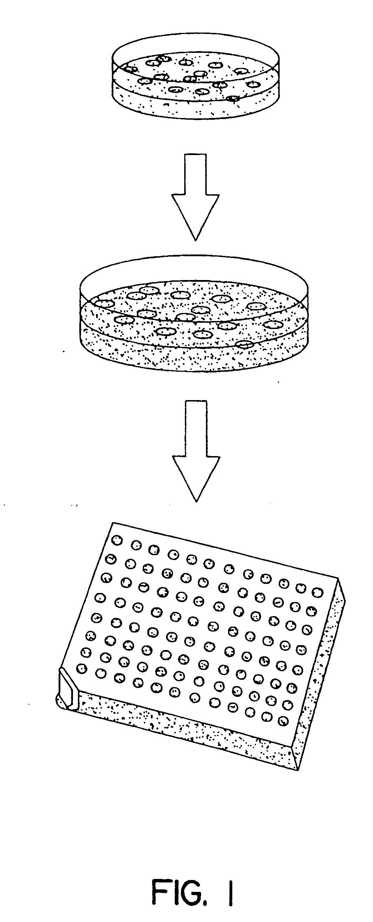

Creation of arrayed cDNA libraries (Figure 1). *E.coli* cDNA libraries were purchased from Invitrogen and were plated at a low density (approximately 1000 clones per plate) onto LB + Amp plates and incubated 1-2 days at 37 °C. Next, 3-4 ml of LB (containing 15% glycerol) was added to each plate, the plate rocked on a platform shaker at low speed, and the LB harvested after resuspension of the colonies in the LB was apparent. A 200 µl portion of the resuspended cells was removed for plasmid DNA isolation and the remaining cells frozen for long-term archival storage at -80 °C.

Plasmid DNA was isolated by means of a kit obtained from Qiagen. Two hundred fifty (250) µlof P2 solution (Qiagen) was added to the 200 µl portion of cells in a 2 ml Eppendorf centrifuge tube. The two solutions were mixed gently and then 250 µl P3 solution (Qiagen) was added and the tube shaken. The mixture was then centrifuged at high speed (14,000 rpm) in an Eppendorf centrifuge. The clarified supernatent (500 µl) was pipetted to a new Eppendorf centrifuge tube and 1 ml of ethanol added to precipitate the DNA. The precipitated DNA was pelleted at high speed (14,000 rpm) for 15 minutes, the ethanol solution decanted off, and the pellet dried *in vacuo.* The pellet was resuspended in 50 µl distilled H₂O and used directly to transform the yeast.

Yeast were transformed using the EZ Yeast Transformation kit (Zymo Research) according to the manufacturer's recommendation, using 2.5 µl DNA, 25 µl competent yeast strain EGY48 and 250 µl of EZ3. The transformed yeast were incubated for 1 hr at 30 °C and the total plated onto SD - trp agar plates. The plates were incubated for an additional 3-4 days at 30 °C, and the cells harvested as for *E.coli* using 3-4 ml SD - trp + 15% glycerol. The harvested yeast from each plate were separately aliquoted into different wells of a deep-dish 96-well plates (the "master library" plates) and frozen at -80 °C for long-term storage.

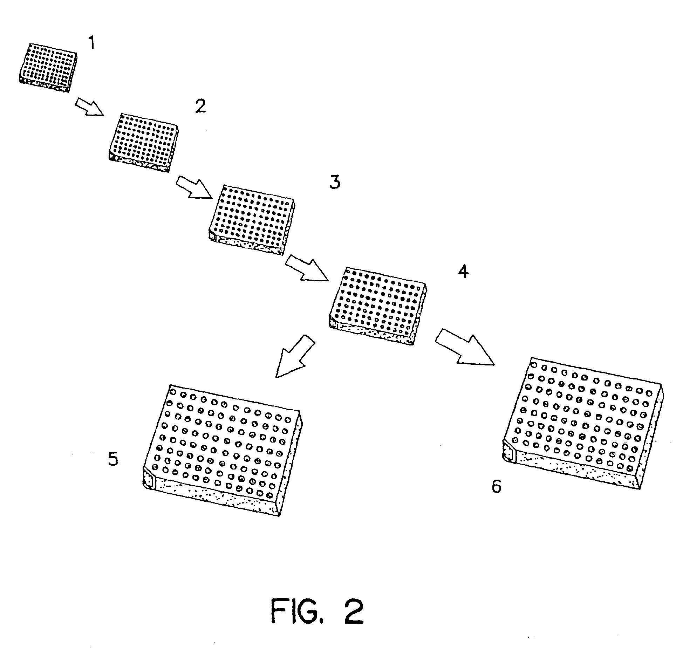

Yeast liquid-mating (Figure 2). Five µl from each of the yeast Master Library well was inoculated into 100 µl of SD - trp or SGal-trp in a 96-well plate and grown overnight at 30 °C. Five µl of each well were transferred to a new 96 well "mating" plate. A 5 µl aliquot of a bait culture (OD₆₀₀ = 1.0) was added to each well along with 10 µl YPD medium. The mating plates were placed into a resealable plastic bag and incubated for 12-36 hr at 30 °C. Each well was then twice serially-diluted 10-fold (final 100 fold dilution) using S-min (- leu, - his, - trp, - ura, + gal, + raff) to a final volume of 110 µl. Alternatively, each well was then diluted 1:10 into S-min, incubated at 30° C for two days, and then diluted 1:40 in S-min (final 400 fold dilution). The diluted matings were incubated for an additional 5-10 days at 30 °C. Ten µl of the mated wells were then transferred to a second set of plates prior to performing the βGal analysis (these mated and out-grown 10 µl stocks ("rescue plates") were later used for rescuing positive clones).

βGal Assay. Cells were lysed by the addition of 100 µl of a solution of Z buffer [Na₂HPO₄, (16.1 g l⁻¹), NaH₂PO₄, (5.5 g l⁻¹), KCI (0.75 g l⁻¹), and MgSO₄, (0.25 g l⁻¹), adjusted to pH 7.0 and sterile-autoclaved] containing oxalyticase (100 U ml⁻¹), SDS (0.1%), and CPRG substrate (2 mg ml⁻¹). The plates were incubated at room temperature until the red βGal chromogenic substrate developed (usually 10 min to 2 hr). To quantitatively measure the wells it was necessary to remove the cell debris by either centrifugation or filtration. The CPRG substrate may be measured at an absorbance of 575 angstrom. Alternatively, β-gal activity was measured using a chemiluminescent substrate. The Tropix Galacton Plus kit was used for this purpose. Twenty-five microliters from each assay well were transferred to the corresponding wells of 96-well luminescence plates. Twenty-five microliters of CL Reaction Buffer (Z buffer containing 0.2% Igepal CA-630, 100 U/ml oxalyticase, and 1% Galacton Plus) was added to each well, and the plates were incubated overnight at room temperature. Fifty microliters of Accelerator II (diluted 1:1 in 0.1 M Na₂CO₃/NaHCO₃, pH 10.5) was added to each well, and the plates were incubated for 5 minutes. Chemiluminescence was then measured in a 96-well luminometer.

Test of pooling sensitivity. A test of the pooled liquid mating strategy was performed using the known strong Y2H interactors RPB4 (yeast polII subunit) and RPB7 (yeast polII subunit) as controls. The RPB4 subunit was subcloned into the activation-domain vector pJG4.5. The recombinant RPB4 fusion was subcloned into the DNA binding domain vector pEG202, transformed into the prey strain and mixed at various percentages (from 0 to 100%) with the same prey strain containing the pJG4.5 parental vector.

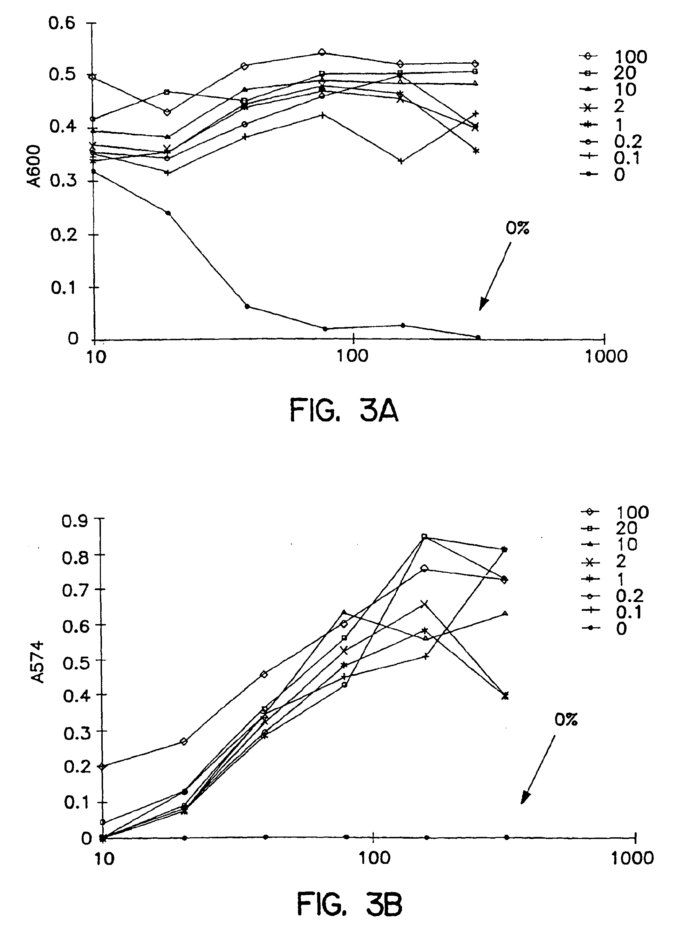

The results (shown in Figure 3) demonstrated that we were able to recover prey strain for this interaction even when the prey initially represented approximately 0.1 % of the prey "mating mixture." The results suggested that dilution of the complex YPD medium approximately 100-fold may be needed in order to see differential growth of the positively-interacting pairs. Dilution of the samples to lower the concentration of YPD complex may be preferable to other methods, such as centrifugation or filtration. This is because dilution is cheaper, faster, and easier to automate. The βGal analysis of the test of the reporter activation in a pooled microtiter plate format showed no significant difference between 0.1 and 100% recombinant fusion at the 100-fold dilution point. At higher dilutions scattering of the βGal activity occured. It may be that at the higher dilutions (of low percentage pools) sampling of positive interactors may be lost.

Test of pooled arrayed cDNA libraries. In the first test of the arrayed-cDNA library experiment, the nuclear receptors RXR and LXRa were tested against ca. 6x10⁵ cDNAs in 6 microtiter plates. Most of the cDNAs were from the commercially-available cDNA libraries derived from human fetal liver (Invitrogen A202-01) and human fetal brain (Invitrogen A212-01).

The cDNA-containing clones were seeded at approximately 1x10³ clones per well. Briefly, the bait strain (containing the target protein, in this case either RXR or LXRa) was added to the cDNA library clones in the wells and mating was allowed to proceed in a complex medium. The mated mixtures were diluted into minimal medium (-leucine) and growth of interactors allowed to take place (growth indicating successful interaction) over 5 or more days. βGal assays were then performed on the wells (see example, Figure 2), and clones from 10 wells exhibiting substantial βGal activity were re-isolated by streaking an aliquot of the library well onto solid minimal medium (-leucine). Plasmids were isolated from those clones and subjected to DNA sequence and bioinformatics analysis. The results are shown below in Table I.

Some of the sequenced clones have been found through traditional Y2H analysis. These include TRIP6 (thyroid receptor interacting protein 6) which has been previously described in the literature from other "standard' interaction-trap experiments against other Nuclear Receptors (it had not yet been tested against LXRa) and TIF1. We believe these represent true-positives. The other clones, both encoding a GCN5 homolog, were isolated twice (in two different wells). We do not yet know if GCN5 homolog is a true or false positive.

Approximately a third of the interacting clones were found to have homology to cDNAs in the Incyte or GenBank databases, but have no ascribed function.

Several clones appear to be known promiscuous positives in interaction trap experiments (namely, cofilin and the heat shock proteins). Now that we know which wells these are in means that they can be eliminated from future analysis. However, it should be noted that when we do exclude these wells, we are also losing information in about the other clones in that well. For example, using RXR as a bait, we found an interaction with thymopoietin-related protein in well 1D9. Yet this same well, when querried with LXRa found a positive interaction with the promiscuous positive HSP90. It is hoped that a large enough library of cDNA is eventually used to obtain redundency in the library analysis.

**Table 1. Results of Y2H Analysis**

| **Bait** | **Sequence ID, well #** | **Representative Homology** | **Comments** |
|---|---|---|---|
| RXR | 5rxr, 1A10 | TIF1 | known NR (+)^{c} |
| LXRa | 13gor4, 3G4 | TRIP6 | known NR (+)^{c} |
| | | | found in standard screen^{e} |
| RXR | 1rxr, 1G6 | annexin (IPP) | found in standard screen^{e} |
| LXRa | 7gor4, 5A10 | GCN5 homolog | involved in transcription^{d} |
| LXRa | 20gor4, 5C5 | GCN5 homolog | involved in transcription |
| RXR | 3rxr, 1D9 | thymopoiten-related | plausable positive |
| LXRa | 12gor4, 2G1 | KIAA0229 | Genbank EST, |
| | | | (no known function) |
| LXRa | 5gor4, 1H8 | Incyte 3122030^{b} | no annotation in GenBank, |
| | | | see also 21gor4 |
| LXRa | 9gor4, 2F8 | Incyte 004215^{b} | no annotation in GenBank |
| LXRa | 15gor4, 4C2 | Incyte 1366945^{b} | no annotation in GenBank |
| LXRa | 21gor4, 2B10 | Incyte 3122030^{b} | no annotation in GenBank |
| RXR | 7rxr, 3B8 | nucleolin gene | known false (+)^{a} |
| LXRa | 1gor4, 1A8 | huHSP86 | known false (+)^{a} |
| LXRa | 3gor4, 1D9 | huHSP90 | known false (+)^{a} |
| LXRa | 11gor4, 5E11 | cofilin | nvolved in cell structure, |
| | | | interacts with actin |

| | | | |
|---|---|---|---|
| ^{a}known common positive in other Y2H screens (E.Golemis). ^{b}No annotation found to genbank database-specific sequence. ^{c}This protein is known to interact with several other nuclear receptors. ^{d}GCN5 possesses histone acetyltransferase (HAT) activity. ^{e}Protein was also isolated using traditional two-hybrid methodology. | | | |

### Example 2

### Open Reading Frame Cloning Strategy

### Cloning open reading frames and use of suppression to control 3' gene fusion

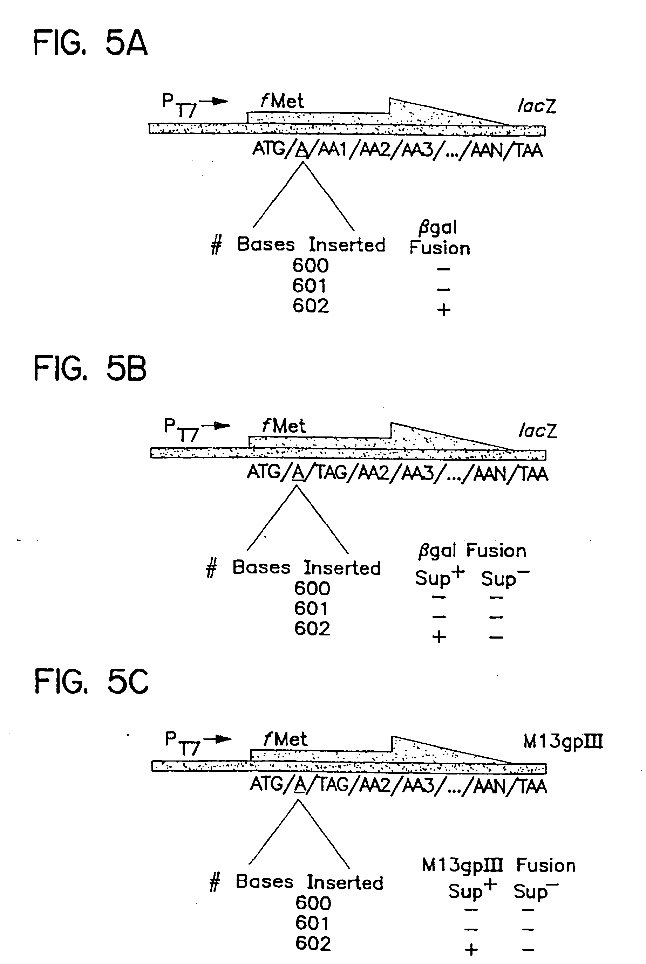

Randomly sheared cDNA of approximately 600 base pairs was isolated and cloned into a frameshifted βgal gene (Figure 5A). Transformed *E.coli* cells that became βgal⁺ contained an open reading frame. In a vector with an amber suppresible termination codon between the 3' end of the cDNA and the 5' end of βgal, the fusion of the cDNA to the βgal was controlled by the Sup phenotype of the *E.coli* strain (Figure 5B). The same type of cloning scheme may be adapted to fuse the 5' end of the M13 phage display protein to the cDNA, in this case viable phage will indicate successful cloning of the open reading frame (Figure 5C).

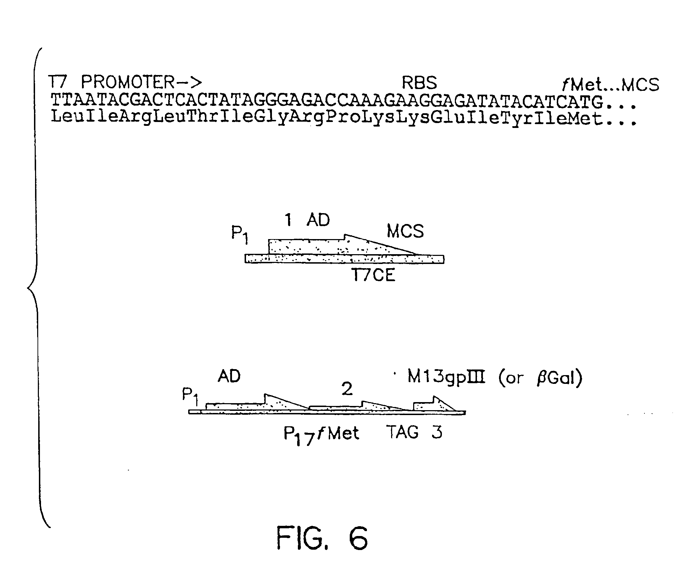

Dynamic recoding of the 3' end of the yeast activation domain. The 3' end of the yeast activation domain was recoded to incorporate the controlling elements for *E.coli* gene expression. Figure 6 is one example of recoding the controlling elements needed for bacteriophage T7 protein expression. The recoded yeast activation domain was then used in conjunction with the open reading frame cloning system to fuse the correct reading frame to the activation domain, and simultaneously to a separate 3' fusion .protein (for example., βgal or M13gp3).

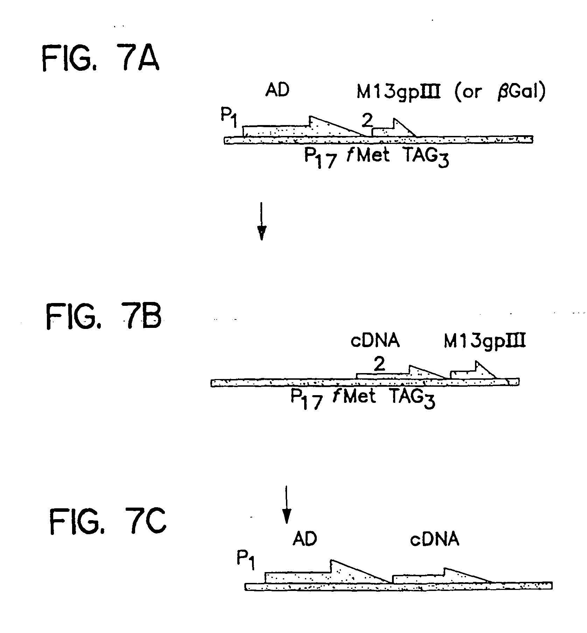

Phenotypic selection in E.coli of cDNA ORFs and concurrent fusion of them to the 3' end of the yeast activation domain and the 5' end of M13gpIII may be performed according to Figure 7: A. Clone random 500 bp cDNA fragments; transform T7RNAP⁺, Sup⁺ *E. coli*; screen plaques. B. Phage Display in E. coli. C. Yeast Two Hybrid in Yeast: transform T7RNAP⁻, Sup⁺ *E. coli*; transform M13 into Sup⁻ yeast.

### BRIEF DESCRIPTION OF DRAWINGS

Figure 1. Creation of arrayed cDNA libraries. *E. coli* cDNA libraries were plated at a low density (approximately 1000 colonies per plate) onto LB + Amp. Next, 3 - 4 ml of LB (containing 15% glycerol) was added to each plate and the LB harvested after resuspension of the colonies in the LB was apparent. Plasmid DNA was isolated by means of a kit obtained from Qiagen (Valencia, CA) and used directly to transform the yeast. The transformed yeast were plated onto SD - trp agar plates. The plates were incubated, and the cells harvested as for *E. coli* using 3 - 4 ml SD - trp + 15% glycerol. The harvested yeast from each plate were separately aliquoted into different wells of deep-dish 96-well plates (the "master library" plates) and frozen at -80°C for long-term storage.
Figure 2. Automatable Y2H format. The following steps were used to perform a Y2H analysis in a microtiter plate: i) Add bait strain to cDNA library strain in a well, ii) allow mating to occur in complex medium, iii) dilute mated mixture into minimal drop-out medium (-leu), iv) allow growth of positively-interacting proteins (growth as readout), v) Perform βGal assays (quantitative readout), vi) sequence (+) clones, query database(s). Legend: 1 = master library; 2 = daughter; 3 = bait added (mated for 24 - 36 hrs); 4 = selective outgrowth (incubate > 5 days); 5 = archive; 6 = βGal.
Figures 3A and 3B. Test of reporter activation in a pooled microtiter plate well. Results are expressed as % of interactor in pool of non-interactors. Known interactors were mixed at a known ratio and tested in the liquid mating format against a bait fusion. X axis: mating dilution; y axis: A₆₀₀ or A₅₇₄. Fig 3A: selective outgrowth after dilution of mated yeast in leucine dropout medium. Activation domain fusion: pJG-scRPB4 (yeast polII subunit); DNA binding domain fusion: pEG-scRPB7 (yeast polII subunit) Fig 3B: βgal assay of wells. Activation domain fusion: pJG-scRPB4 (yeast polII subunit); DNA binding domain fusion: pEG-scRPB7 (yeast poIII subunit).

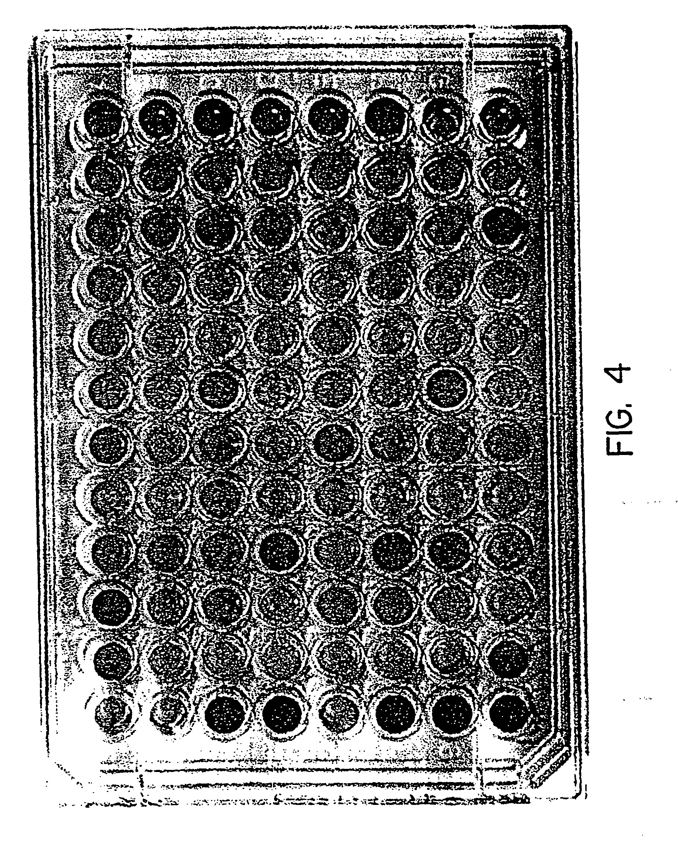

Figure 4. Y2H analysis of the human Nuclear Receptor RXR screened against (approximately) 88 x 10⁴ cDNA clones. Each well of the three 96-well plate shown represents a βgal assay performed on a pEG202 RXR bait plasmid-containing yeast strain mated to approximately 1000 yeast clones of a pJG4.5 AD library. The eight left-most wells in each plate are positive and negative controls, (from top to bottom of the plate); a) pEG202 x pJGRXR, b) pEG202, c) pEGKREV1 x pJGRAF, d) pEGKREV1 x pJGKRIT1, e) pEGRAS x pJGKRIT1, f) pEGRAS x pJGRAF, g) pEGRXR x pJGGOR4, and h) pEGGOR4 x pJGRXR.
Figure 5. Open reading frame cloning strategy. Cloning Open Reading Frames. A. Frameshift fusion to βgal⁺. Randomly sheared cDNA of approximately 600 base pairs were isolated and cloned into a frameshifted βgal gene. Transformed *E.coli* cells that became β gal⁺ contained an open reading frame. B. Frameshift fusion to βgal⁺ in a Sup⁺ host. In a vector with an amber suppresible termination codon between the 3' end of the cDNA and the 5' end of βgal, the fusion of the cDNA to the βgal was controlled by the Sup phenotype of the *E.coli* strain. C. Frameshift fusion to M13gpIII in a Sup⁺ host. The same type of cloning scheme may be adapted to fuse the 5' end of the M13 phage display protein to the cDNA, in this case viable phage will indicate successful cloning of the open reading frame.
Figure 6. Dynamic recoding of the 3' yeast activation domain. Translation of the T7 controlling elements (T7CE). The 3' end of the yeast activation domain (AD) was recoded to incorporate the controlling elements for *E.coli* gene expression. Shown is one example of recoding the controlling elements needed for bacteriophage T7 protein expression. The recoded yeast activation domain was then used in conjunction with the open reading frame cloning system to fuse the correct reading frame to the activation domain, and simultaneously to a separate 3' fusion protein (for example, βgal or M13gp3). Legend: 1 = yeast; 2 = cDNA insert; 3 = frameshift.
Figure 7. Phenotypic selection in. *E. coli* of cDNA Open Reading Frames and concurrent fusion of them to the 3' end of the yeast activation domain (AD) and the 5' end of M13gpIII. A. clone random 500 bp cDNA fragments; transform T7RNAP⁺, Sup⁺ *E. coli;* screen plaques. B. Phage Display in *E*. *coli.* C. Yeast Two Hybrid in Yeast: transform T7RNAP⁻, Sup⁺ *E.* coli; transform M 13 into Sup⁻ yeast. Legend: 1 = yeast; 2 = insert site; 3 = frameshift.

## Claims

1. A method for detecting an interaction between a first test protein and a second test protein, comprising:
(a) providing a yeast host cell containing a reporter gene wherein the reporter gene expresses a detectable protein when the reporter gene is activated by an amino acid sequence including a transcriptional activation domain when the transcriptional activation domain is in sufficient proximity to the reporter gene;
(b) providing a first chimeric gene that is expressed in the yeast host cell, the first chimeric gene comprising a DNA sequence that encodes a first hybrid protein, the first hybrid protein comprising:
(i) a DNA-binding domain that recognizes a binding site on the reporter gene in the host cell; and
(ii) a first test protein or fragment thereof that is to be tested for interaction with at least one second test protein or fragment thereof;
(c) providing a second chimeric gene that is expressed in the yeast host cell, the second chimeric gene comprising a DNA sequence that encodes a second hybrid protein, the second hybrid protein comprising:
(i) the transcriptional activation domain; and
(ii) a second test protein or fragment thereof that is to be tested for interaction with the first test protein or fragment thereof; wherein interaction between the first test protein and the second test protein in the yeast host cell causes the transcriptional activation domain to activate transcription of the reporter gene;
(d) introducing the second chimeric gene into a yeast host cell strain of a first mating type and subsequently introducing said cells into an arraying means thereby creating a master library plate;
(e) introducing cells from the master library plate into a second arraying means which is an arraying means for liquid media thereby creating a mating set;
(f) introducing the first chimeric gene into a yeast host cell strain of the opposite mating type to (d) and subsequently introducing said cell into the mating set thereby allowing mating to occur in liquid medium; and
(g) selecting for outgrowth of the interaction of the first and second genes.

2. A method for detecting an interaction between a first test protein and a second test protein according to claim 1, comprising the additional steps:
(h) removing a portion of the mating set to a third arraying means thereby creating a rescue set;
(i) determining whether the reporter gene has been expressed in the mating set; and
(j) analyzing the cells from the rescue plate.

3. The method according to Claim 2 wherein the host cell is *Saccharomyces cerevisiae.*

4. The method according to Claim 2 wherein the reporter gene is selected from the group consisting of LEU2, IacZ, HIS3, URA3, LYS2, GAL1, *E. coli* galK, GFP, CUP1, CAT, G418 and GUS.

## Patentansprüche

1. Verfahren zum Ermitteln einer Interaktion zwischen einem ersten Test-Protein und einem zweiten Test-Protein, das umfasst:
(a) das Bereitstellen einer Hefe-Wirtszelle, die ein Reporter-Gen enthält, wobei das Reporter-Gen ein nachweisbares Protein exprimiert, wenn das Reporter-Gen von einer Aminosäuresequenz, die eine Transkriptions-Aktivierungsdomäne enthält, aktiviert wird, wenn die Transkriptions-Aktivierungsdomäne in ausreichender Nähe zu dem Reporter-Gen ist;
(b) das Bereitstellen eines ersten chimären Gens, das von der Hefe-Wirtszelle exprimiert wird, wobei das erste chimäre Gen eine DNA-Sequenz umfasst, die ein erstes Hybrid-Protein codiert, wobei das erste Hybrid-Protein umfasst:
(i) eine DNA-bindende Domäne, die eine Bindestelle auf dem Reporter-Gen in der Wirtszelle erkennt; und
(ii) ein erstes Test-Protein oder Fragment davon, das auf Interaktion mit mindestens einem zweiten Test-Protein oder Fragment davon getestet werden soll;
(c) das Bereitstellen eines zweiten chimären Gens, das in der Hefe-Wirtszelle exprimiert wird, wobei das zweite chimäre Gen eine DNA-Sequenz umfasst, die ein zweites Hybrid-Protein codiert; wobei das zweite Hybrid-Protein umfasst:
(i) die Transkriptions-Aktivierungsdomäne;
(ii) ein zweites Test-Protein oder Fragment davon, das auf Interaktion mit dem ersten Testprotein oder Fragment davon getestet werden soll; wobei Interaktion zwischen dem ersten Test-Protein und dem zweiten Test-Protein in der Hefe-Wirtszelle die Transkriptions-Aktivierungsdomäne veranlasst, die Transkription des Reporter-Gens zu aktivieren;
(d) das Einführen des zweiten chimären Gens in einen Hefe-Wirtszellstamm eines ersten Paarungstyps und das anschließende Einführen der Zellen in eine Array-Vorrichtung, wobei eine Master-Bibliotheksplatte erstellt wird;
(e) das Einführen von Zellen von der Master-Bibliotheksplatte in eine zweite Array-Vorrichtung, die eine Array-Vorrichtung für flüssiges Medium ist, wobei ein Paarungssatz erstellt wird;
(f) das Einführen des ersten chimären Gens in einen Hefe-Wirtszellstamm des entgegengesetzten Paarungstyps zu (d) und anschließend das Einführen der Zelle in den Paarungssatz, wodurch es ermöglicht wird, dass die Paarung in flüssigem Medium erfolgt; und
(g) das Selektieren nach Auswuchs der Interaktion der ersten und zweiten Gene.

2. Verfahren zum Nachweisen einer Interaktion zwischen einem ersten Test-Protein und einem zweiten Test-Protein gemäß Anspruch 1, das die zusätzlichen Schritte umfasst:
(h) das Entfernen eines Teils des Paarungssatzes zu einer dritten Array-Vorrichtung, wobei ein Rettungssatz erstellt wird;
(i) das Ermitteln, ob das Reporter-Gen in dem Paarungssatz exprimiert wurde; und
(j) das Analysieren der Zellen von der Rettungsplatte.

3. Verfahren gemäß Anspruch 2, wobei die Wirtszelle *Saccharomyces cerevisiae* ist.

4. Verfahren nach Anspruch 2, wobei das Reporter-Gen ausgewählt ist aus der Gruppe bestehend aus LEU2, lacZ, HIS3, URA3, LYS2, GAL1, *E. coli-*galK, GFP, CUP1, CAT, G418 und GUS.

## Revendications

1. Méthode pour détecter une interaction entre une première protéine d'essai et une seconde protéine d'essai, comprenant les étapes consistant :
(a) à fournir une cellule hôte de levure contenant un gène rapporteur dans laquelle le gène rapporteur exprime une protéine détectable lorsque le gène rapporteur est activé par une séquence d'aminoacides comprenant un domaine d'activation de transcription lorsque le domaine d'activation de transcription est suffisamment proche du gène rapporteur ;
(b) à fournir un première gène chimère qui est exprimé dans la cellule hôte de levure, le premier gène chimère comprenant une séquence d'ADN qui code pour une première protéine hybride, la première protéine hybride comprenant :
(i) un domaine de liaison d'ADN qui reconnaît un site de liaison sur le gène rapporteur dans la cellule hôte ; et
(ii) une première protéine d'essai ou un de ses fragments qui doit être testé pour l'interaction avec au moins une seconde protéine d'essai ou un de ses fragments ;
(c) à fournir un second gène chimère qui est exprimé dans la cellule hôte de levure, le second gène chimère comprenant une séquence d'ADN qui code pour une seconde protéine hybride, la seconde protéine hybride comprenant :
(i) le domaine d'activation de transcription ; et
(ii) une seconde protéine d'essai ou un de ses fragments qui doit être testé pour l'interaction avec la première protéine d'essai ou un de ses fragments ; l'interaction entre la première protéine d'essai et la seconde protéine d'essai dans la cellule hôte de levure amenant le domaine d'activation de transcription à activer la transcription du gène rapporteur ;
(d) à introduire le second gène chimère dans une souche d'une cellule hôte de levure d'un premier type de conjugaison et à introduire ensuite lesdites cellules dans un moyen de formation de réseau, en créant ainsi une plaque de banque mère ;
(e) à introduire les cellules provenant de la plaque de banque mère dans un second moyen de formation de réseau, qui est un moyen de formation de réseau pour milieux liquides, en créant ainsi un ensemble de conjugaison ;
(f) à introduire le premier gène chimère dans une souche de cellule hôte de levure du type de conjugaison opposé à (d) et ensuite à introduire ladite cellule dans l'ensemble de conjugaison, ce qui permet à la conjugaison de se produire dans le milieu liquide ; et
(g) à sélectionner le résultat de croissance de l'interaction des premier et second gènes.

2. Méthode pour détecter une interaction entre une première protéine d'essai et une seconde protéine d'essai suivant la revendication 1, comprenant les étapes supplémentaires consistant :
(h) à prélever une partie de l'ensemble de conjugaison et à l'amener à un troisième moyen de formation de réseau, en créant ainsi un ensemble de sauvetage ;
(i) à déterminer si le gène rapporteur a été exprimé dans l'ensemble de conjugaison ; et
(j) à analyser les cellules provenant de la plaque de sauvetage.

3. Méthode suivant la revendication 2, dans laquelle la cellule hôte est *Saccharomyces cerevisiae.*

4. Méthode suivant la revendication 2, dans laquelle le gène rapporteur est choisi dans le groupe consistant en LEU2, LacZ, HIS3, URA3, LYS2, GAL1, galK de *E. coli,* GFP, CUP1, CAT, G418 et GUS.
